# EUROPÄISCHE PATENTSCHRIFT

(11) **EP 1 108 403 B1**
(45) Veröffentlichungstag und Bekanntmachung des Hinweises auf die Patenterteilung: **26.08.2009**
(21) Anmeldenummer: 00811071.0
(22) Anmeldetag: 14.11.2000
(51) Int. Cl.: A61F 2/38

(54) **Bausatz für eine Kniegelenkprothese**
Assembly set for a knee prosthesis
Jeu de pièces pour l'assemblage d'une prothèse de genou

(30) Priorität: 13.12.1999 EP 99811151
(43) Veröffentlichungstag der Anmeldung: 20.06.2001
(73) Patentinhaber: Zimmer GmbH, 8404 Winterthur (CH)
(72) Erfinder: Leclercq, Vincent, 8404 Winterthur (CH)
(74) Vertreter: Manitz, Finsterwald & Partner GbR

(56) Entgegenhaltungen:
- EP-A- 0 553 585
- EP-A- 0 913 132
- WO-A-99/13804
- US-A- 4 950 297

## Beschreibung

Die Erfindung betrifft einen Bausatz für eine Kniegelenkprothese gemäss dem unabhängigen Patentanspruch 1.

Kniegelenkprothesen sind in grosser Zahl bereits auf dem Markt erhältlich. Die Tendenz geht dabei mehr und mehr dahin, die Prothesen modular zu gestalten, um dem Arzt die Gelegenheit zu geben, gegebenenfalls noch intraoperativ die tatsächlichen anatomischen Gebenheiten eines Patienten (z.B. Qualität des Knochens, Qualtität der Bänder, etc.) optimal berücksichtigen zu können bei der Auswahl des Prothesentyps.

Bei Kniegelenkprothesen, welche ein Tibiateil umfassen, bei welchem auf der dem Femur zugewandten Fläche ein Meniskusteil vorgesehen ist, sind grundsätzlich folgende vier Typen in Bezug auf die Art der Beweglichkeit des Meniskusteils relativ zu der dem Femur zugewandten Fläche des Tibiateils zu unterscheiden: Prothesen mit einem relativ zu dieser Fläche des Tibiateils unbeweglich angeordneten Meniskusteil, Prothesen mit einem relativ zu dieser Fläche des Tibiateils ausschliesslich drehbar angeordneten Meniskusteil, Prothesen mit einem relativ zu dieser Fläche des Tibiateils ausschliesslich translatorisch verschiebbar angeordneten Meniskusteil, sowie Prothesen mit einem relativ zu dieser Fläche des Tibiateils sowohl drehbar als auch translatorisch verschiebbar angeordneten Meniskusteil.

Während die einzelnen Typen solcher Kniegelenkprothesen - für sich genommen - bereits bekannt sind, so ist es doch zumindest so, dass diejenigen Meniskusteile, die relativ zu der dem Femur zugewandten Fläche des Tibiateils unbeweglich angeordnet sind, regelmässig fest und unlösbar oder nur sehr schwer lösbar mit dem Tibiateil verbunden sind, z.B. mit Hilfe von Schnappverbindungen, wie dies in der EP-A-0,923,916 gezeigt ist. Eine Prothese, bei welcher das Meniskusteil relativ zu der dem Femur zugewandten Fläche des Tibiateils ausschliesslich translatorisch verschiebbar angeordnet ist, ist beispielsweise aus der EP-A-0,913,132 bekannt. Eine Prothese, bei welcher das Meniskusteil relativ zu der dem Femur zugewandten Fläche des Tibiateils sowohl drehbar als auch translatorisch verschiebbar angeordnet ist, ist beispielsweise aus der EP-A-0,519,873 bekannt.

Nachteilig ist jedoch, dass wenigstens für diejenigen Prothesen, bei denen das Meniskusteil fest mit dem Tibiateil verbundenen ist, z.B. mittels der Schnappverbindungen, und für diejenigen Prothesen, bei denen das Meniskusteil - in welcher Art und Weise auch immer - relativ zu dem Tibiateil beweglich ist, unterschiedliche Tibiateile benötigt werden. Das Tibiateil für das unbewegliche Meniskusteil muss zudem zusätzliche Mittel zum Befestigen des Meniskusteils aufweisen (z.B. Vorsprünge, die mit entsprechenden Vorsprüngen am Meniskusteil die Schnappverbindung eingehen), was die Herstellung des Tibiateils und des Meniskusteils aufwendiger macht und auch oft dazu führt, dass das Tibiaplateau des Tibiateils, auf welchem das Meniskusteil aufliegt, dünner wird. Ausserdem ist auch das Meniskusteil an seiner Peripherie, also dort, wo es über den Vorsprüngen (zur Bildung der Schnappverbindung) des Tibiateils zu liegen kommt, dünner ausgebildet,, was einerseits herstellungstechnisch aufwendiger ist und andererseits das Meniskusteil in diesen Bereichen zum Teil extremen Belastungen aussetzt.

Aus WO 99/13804 A1 sind Kniegelenks endoprothesen bekannt, bei denen die Art der Beweglichkeit zwischen Tibiateil und Meniskusteil durch die Auswahl eines Führungselements festlegbar ist.

Es ist eine Aufgabe der Erfindung, einen Bausatz für eine Kniegelenkprothese vorzuschlagen, welcher die intraoperative Flexibilität bezüglich des zu implantierenden Typs der Kniegelenkprothese für den operierenden Arzt aufrecht erhält, gleichzeitig aber für jeden in Frage kommenden Typ eine definierte Führung des Meniskusteil gewährleistet.

Diese Aufgabe wird durch einen Bausatz für eine Kniegelenkprothese gelöst, wie er durch die Merkmale des unabhängigen Patentanspruchs charakterisiert ist. Besonders vorteilhafte Ausgestaltungen des erfindungsgemässen Bausatzes ergeben sich aus den abhängigen Patentansprüchen.

Der erfindungsgemässe Bausatz umfasst mehrere Führungselemente, die so ausgebildet sind, dass das jeweilige Führungselement bei zusammengesetzter Prothese mit dem Tibiateil und mit dem Meniskusteil in Eingriff ist und die Bewegbarkeit des Meniskusteils relativ zum Tibiateil festlegt. Somit ist es - bei gegebenem Meniskusteil - möglich, durch die Auswahl eines entsprechenden Führungselements festzulegen, ob das Meniskusteil bei zusammengesetzter Prothese relativ zum Tibiateil unbeweglich angeordnet ist, oder ob das Meniskusteil relativ zum Tibiateil beweglich angeordnet ist, also auf dem Tibiaplateau bewegt werden kann. Dabei kann die Bewegbarkeit auf dem Tibiaplateau bedeuten, dass das Meniskusteil entweder ausschliesslich drehbar ist, oder ausschliesslich translatorisch verschiebbar, oder sowohl drehbar als auch translatorisch verschiebbar.

Darüberhinaus wird für die verschiedenen Typen von Kniegelenkprothesen auch nur noch ein Typ von Tibiateil benötigt (allenfalls noch verschiedene Grössen, jedoch nicht mehr verschiedene Typen), weil die Bewegbarkeit eben durch das Führungselement festgelegt wird. Dadurch wird der Aufwand bei der Fertigung erheblich verringert, weil einerseits keine verschiedenen Typen von Tibiateilen hergestellt werden müssen. Darüberhinaus wird auch der fertigungstechnische Aufwand für Vorsprünge etc. für das Befestigen von Meniskusteilen mittels Schnappverbindung oder ähnlichem vermieden. Gleiches gilt für die Meniskusteile, es müssen also keine besonderen Meniskusteile hergestellt werden für eine Kniegelenkprothese, bei welcher das Meniskusteil relativ zum Tibiateil unbeweglich sein soll. Auch die übrigen bis anhin aufgetretenen Nachteile (geringere Dicke des Tibiaplateaus bzw. zum Teil extreme Belastung in den peripheren Bereichen des Meniskusteils) fallen dahin.

Gleichzeitig bleibt jedoch die intraoperative Flexibilität für den operierenden Arzt im Hinblick auf den Typ der zu implantierenden Kniegelenkprothese vollständig erhalten. Der Arzt kann nämlich - entsprechend den tatsächlichen anatomischen Gegebenheiten - nötigenfalls noch intraoperativ entscheiden, dass ein anderer Typ von Kniegelenkprothese als der präoperativ geplante Typ für den Patienten die optimale Versorgung darstellt, ohne dass zusätzliche - nicht in dem Bausatz enthaltene - Prothesenteile bereitgestellt werden müssen.

Bei dem erfindungsgemässen Bausatz weist das Tibiateil in seiner dem Femurteil zugewandten Fläche eine Bohrung auf, die mit einer Aussparung versehen ist. Die einzelnen Führungselemente weisen jeweils einen Zapfen auf, der bei zusammengesetzter Prothese in diese Bohrung des Tibiateils hineinragt. Ausserdem weisen die Führungselemente jeweils einen Lenker auf, der bei zusammengesetzter Prothese in Eingriff mit einer entsprechenden Führungsfläche am Meniskusteil ist. Manche Zapfen weisen einen Vorsprung auf, der in der Aussparung der Bohrung zu liegen kommt, während andere Zapfen keinen solchen Vorsprung aufweisen.

Diejenigen Führungselemente, die einen Zapfen mit einem Vorsprung aufweisen, sind nach dem Einbringen des Zapfens in die Bohrung des Tibiateils dort drehfest gelagert. Das gilt auch für den zu dem jeweiligen Führungselement gehörenden Lenker und damit auch für das mit dem Lenker in Eingriff befindliche Meniskusteil. Hingegen sind diejenigen Führungselemente, welche keinen solchen Vorsprung aufweisen, drehbar gelagert, was entsprechend auch für den Lenker und damit auch für das mit dem Lenker in Eingriff befindliche Meniskusteil gilt. Auf die Möglichkeit der translatorischen Verschiebung des Meniskusteils entlang dem Lenker wird weiter unten noch eingegangen.

Bei einem vorteilhaften Ausführungsbeispiel weist das Meniskusteil ein Langloch auf, in welchem der Lenker bei zusammengesetzter Prothese aufgenommen wird. Dieses Ausführungsbeispiel zeichnet sich insofern aus, als es während der Operation besonders einfach zusammengesetzt werden kann. Zunächst werden nämlich üblicherweise das Tibiateil an der präparierten Tibia und das Femurteil am präparierten Femur befestigt. Dann kann- je nach Prothesentyp - ein entsprechendes Führungselement in die Bohrung des Tibiateils eingesetzt werden. Ist dies erfolgt, so kann anschliessend das Meniskusteil über den Lenker des Führungselements geschoben werden und schliesslich das Kniegelenk reponiert werden.

Besonders vorteilhaft ist es, wenn Führungselemente mit unterschiedlicher Länge des Lenkers in dem Bausatz vorgesehen sind, sodass bei ein und demselben Meniskusteil je nach Länge des Lenkers bei zusammengesetzter Prothese eine translatorische Bewegung des Meniskusteils möglich ist oder nicht. Das heisst, dass für ein gegebenes Meniskusteil (z.B. mit vorgegebener Länge des Langlochs) alleine durch die Wahl des Führungselements festgelegt werden kann, ob eine translatorische Verschiebung des Meniskusteils am Lenker entlang zugelassen werden soll oder nicht. Diese Entscheidung ist oft abhängig vom Zustand des Bandapparats. Soll eine solche translatorische Verschiebung zugelassen werden, so ist die Länge des Lenkers kürzer zu wählen als die Länge des Langlochs. Soll hingegen keine translatorische Verschiebung des Meniskusteils am Lenker entlang zugelassen werden, so ist die Länge des Lenkers gleich zu wählen wie die Länge des Langlochs.

Bei einem weiteren vorteilhaften Ausführungsbeispiel des erfindungsgemässen Bausatzes ist der Zapfen des Führungselements als Hohlzapfen ausgebildet zur Aufnahme eines Zapfens eines Stabilisierungselementes. Dieses Stabilisierungselement ragt bei zusammengesetzter Prothese zwischen die Kondylen des Femurteils hinein und weist Mittel zur Varus-Valgus-Stabilisierung auf. Diese Mittel zur Varus-Valgus-Stabilisierung können z.B. Stabilisierungsflächen sein, welche mit entsprechenden Gegenflächen zusammenwirken. Andererseits kann auch nur das Führungselement mit dem Hohlzapfen alleine verwendet werden, sodass ein derartig ausgebildetes Führungselement optional zwar die Aufnahme eines solchen Stabilisierungselements gestattet, aber ansonsten genau gleich funktioniert wie ein Führungselement, bei welchem der Zapfen als Vollzapfen ausgebildet ist.

Weitere vorteilhafte Ausgestaltungen des erfindungsgemässen Bausatzes ergeben sich aus der nachfolgenden Beschreibung eines Ausführungsbeispiels anhand der Zeichung.

Es zeigen, teilweise in schematischer Darstellung und/oder im Schnitt:
- Fig. 1: ein Ausführungsbeispiel einer Kniegelenkprothese, welche mit Hilfe des erfindungsgemässen Bausatzes zusammengestellt werden kann, in Explosionsdarstellung,
- Fig. 2: ein Ausführungsbeispiel eines Tibiateils mit aufgesetztem Meniskusteil und mit einem Führungselement, welches zwischen Tibiateil und Meniskusteil angeordnet ist und mit beiden im Eingriff ist
- Fig. 3: eine Ansicht von unten auf das Führungselement gemäss Fig. 3
und
- Fig. 4: eine Ansicht von unten auf ein alternatives Führungselement.

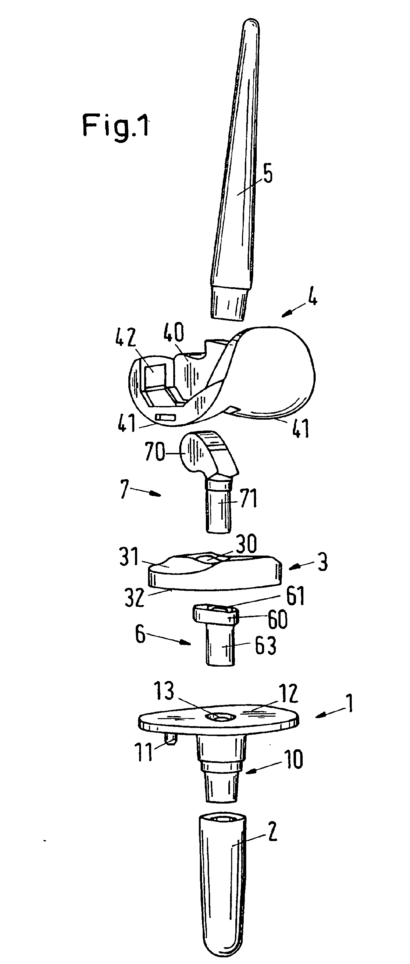

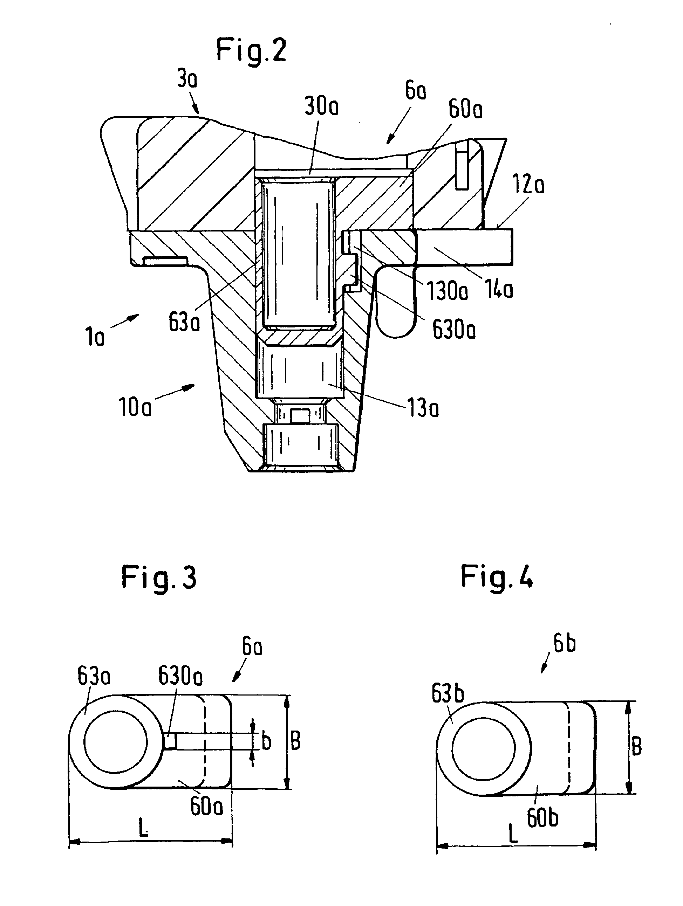

In der in Fig. 1 gezeigten Explosionsdarstellung eines Ausführungsbeispiels eines speziellen Typs einer Kniegelenkprothese, welche mit einem erfindungsgemässen Bausatz zusammengestellt werden kann, erkennt man ein Tibiateil 1, welches an seinem Ansatzstück 10 mit einem Verankerungsschaft 2 versehen werden kann, sofern dies von der Beschaffenheit der Tibia her erforderlich ist, was insbesondere bei Revisionsoperationen geboten sein kann. An der Unterseite des Tibiateils 1 sind z.B. zwei Verankerungsstifte 11 vorgesehen, von denen in Fig. 1 nur einer zu erkennen ist. Diese Verankerungsstifte 11 werden in entsprechend erstellte Bohrungen in der Tibia eingebracht und verhindern ein Verdrehen des Tibiateils 1 gegenüber der Tibia. Aus zeichnerischen Gründen wurde bei der Darstellung des Tibiateils 1 auf die Darstellung einer Aussparung für das hintere Kreuzband verzichtet, diese kann jedoch standardmässig im Tibiateil 1 vorgesehen sein (siehe Fig. 2), sodass das Tibiateil 1 unabhängig von dem Zustand der Bänder universell einsetzbar ist.

Ferner erkennt man in Fig. 1 ein Femurteil 4, welches mit einem Verankerungsschaft 5 versehen werden kann, sofern dies von der Beschaffenheit des Femurs her erforderlich ist, was insbesondere bei Revisionsoperationen geboten sein kann. Das Femurteil 4 weist bei diesem Ausführungsbeispiel einen Stabilisierungskasten 40 auf, welcher zwischen zwei Kondylen 41 angeordnet ist. Ferner erkennt man Zementtaschen 42, in welchen sich bei einer zementierten Implantation des Femurteils 4 Knochenzement für die Befestigung des Femurteils 4 am Femur befindet.

Weiterhin ist in Fig. 1 ein Meniskusteil 3 zu erkennen, welches zwischen dem Femurteil 4 und dem Tibiateil 1 angeordnet ist. Das Meniskusteil 3 weist ein Langloch 30 auf sowie zwei Lagerschalen 31, von denen in Fig. 1 nur eine zu erkennen ist. In den Lagerschalen 31 des Meniskusteils 3 kommen bei zusammengesetzter Prothese die Kondylen 41 des Femurteils 4 zu liegen.

Die dem Femurteil 4 zugewandte Fläche 12 des Tibiateils 1, bei gleitenden Meniski oft auch als Gleitfläche bezeichnet, ist eben ausgebildet und typischerweise geschliffen und/oder poliert. Auch die Unterseite 32 des Meniskusteils 3 ist eben und glatt ausgebildet, sodass grundsätzlich sowohl eine translatorische als auch eine rotatorische Bewegung des Meniskusteils 3 relativ zum Tibiateil 1 möglich ist. Das Tibiateil 1 weist ausserdem in der dem Femurteil 4 zugewandten Fläche 12 eine Bohrung 13 auf.

Weiterhin erkennt man in Fig. 1 ein Führungselement 6, welches einen Zapfen 63 aufweist, welcher bei zusammengesetzter Prothese in die Bohrung 13 des Tibiateils 1 hineinragt. Ferner weist das Führungselement 6 einen Lenker 60 auf, welcher bei zusammengesetzter Prothese in dem Langloch 30 des Meniskusteils 3 aufgenommen wird. Im übrigen ist zu erkennen, dass der Zapfen 63 des Führungselements als Hohlzapfen ausgebildet ist, er weist nämlich eine Bohrung 61 auf, in welcher bei diesem Ausführungsbeispiel bei zusammengesetzter Prothese der Zapfen 71 eines Stabilisierungselements7 aufgenommen wird.

Das Stabilisierungselement 7 ragt bei diesem Ausführungsbeispiel bei zusammengesetzter Prothese zwischen die Kondylen 41 des Femurteils 4 in den Stabilisierungskasten 40 hinein. Das Stabilisierungselement 7 weist dort zwei Stabilisierungsflächen 70 auf, welche zusammen mit den inneren Seitenwänden des Stabilisierungskastens 40 eine Varus-Valgus-Stabilisierung bewirken.

Fig. 2 zeigt ein Ausführungsbeispiel eines Tibiateils 1a, bei welchem standardmässig eine Aussparung 14a für das hintere Kreuzband vorgesehen ist. Das Tibiateil 1a weist ebenfalls ein Ansatzstück 10a auf, in welchem gegebenenfalls ein Verankerungsschaft (in Fig. 2 nicht dargestellt) aufgenommen werden kann. Ferner erkennt man das Meniskusteil 3a, welches auf der dem Femurteil (nicht dargestellt) zugewandten Fläche 12a des Tibiateils 1a angeordnet ist.

Weiterhin erkennt man in Fig. 2 das Führungselement 6a, welches einen Zapfen 63a aufweist, der in die Bohrung 13a des Tibiateils 1a hineinragt. Der Zapfen 63a des Führungselements 6a ist hier als Hohlzapfen ausgebildet. Er weist eine Bohrung 61 a auf, in welcher beispielsweise ein Stabilisierungselement (siehe Fig. 1 ) aufgenommen werden kann. Die tatsächliche Aufnahme eines Stabilisierungselements ist jedoch nur optional, das Führungselement 6a kann ebensogut für andere Prothesentypen ohne ein solches Stabilisierungsstück verwendet werden.

Der Lenker 60a des Führungselements 6a ist in dem Langloch 30a des Meniskusteils 3a aufgenommen, und zwar so, dass der Lenker im Eingriff mit mindestens einer Führungsfläche am Meniskusteil ist (hier mit den Seitenwänden des Langlochs). Bei diesem Ausführungsbeispiel ist das Langloch 30a abgestuft ausgebildet, sodass das Führungselement 30a keinesfalls nach oben durch das Langloch 30a herausgleiten kann, jedoch das Meniskusteil 3a trotzdem von oben her über den Lenker 60a geschoben werden kann.

Man erkennt in Fig. 2, dass die Länge L des Lenkers 60a (siehe Fig. 3) und die Länge des Langlochs 30a (zumindest in dem Teil des Langlochs, in welchem der Lenker zu liegen kommt) gleich sind. Das hat zur Folge, dass das Meniskusteil 3a nicht entlang des Lenkers 60a verschoben werden kann. Eine translatorische Verschiebung des Meniskusteils 3a auf dem Tibiateil 1a entlang des Lenkers 60a ist somit ausgeschlossen.

Der Zapfen 63a weist ferner einen Vorsprung 630a auf, welcher in einer Aussparung 130a der Bohrung 13a im Tibiateil 1a zu liegen kommt. Die Breite b (Fig. 3) dieses Vorsprungs 63a ist auf die Breite der Aussparung 130a abgestimmt. Beim Einbringen des Zapfens 63a wird dieser so in die Bohrung 13a des Tibiateils 1 a eingebracht, dass der Vorsprung 630a am Zapfen 63a in die Aussparung 130a der Bohrung 13a hineingleitet. Danach ist der Zapfen 63a gegenüber dem Tibiateil 1 a drehfest gelagert.

Wird nun das Meniskusteil 3a mit seinem Langloch 30a von oben her über den Lenker 60a des Führungselements 6a geschoben, so kann anschliessend auch das Meniskusteil 3a gegenüber dem Tibiateil 1a nicht verdreht werden, weil die Breite B (siehe Fig. 3) des Führungselements 6a auf die Breite des Langlochs 30a abgestimmt ist. Eine translatorische Verschiebung entlang des Lenkers 60a scheidet ebenfalls aus, weil die Länge L des Lenkers 60a auf die Länge des Langlochs 30a abgestimmt ist.

Bei dem in Fig. 2 gezeigten Ausführungsbeispiel ist also weder eine translatorische noch eine rotatorische Bewegbarkeit des Meniskusteils 3a relativ zum Tibiateil 1a möglich. Das Meniskusteil 3a ist vielmehr gegenüber dem Tibiateil 1a unbeweglich angeordnet.

Dennoch ist es möglich, mit dem gleichen Tibiateil 1a auch andere Prothesentypen zusammenzustellen, bei denen das Meniskusteil 3a auf unterschiedliche Art relativ zum Tibiateil 1a beweglich ist. Hierzu betrachte man im folgenden das Führungselement 6a in Fig. 3 bzw. ein alternatives Führungselement 6b in Fig. 4, welche beide in einer Ansicht von unten dargestellt sind. Dabei soll die Annahme gelten, dass wenigstens die Abmessungen des Langlochs des Meniskusteils unverändert bleiben, wenn nicht sogar das ganze Meniskusteil.

Betrachtet man zunächst das Führungselement 6a in Fig. 3, so erkennt man, dass das Führungselement 6a gegenüber dem Tibiateil 1a mit Hilfe des Vorsprungs 630a am Zapfen 63a drehfest gelagert ist, wie vorstehend bereits beschrieben. Wird nun ein Lenker mit einer kürzeren Länge L aber mit gleicher Breite B wie vorher gewählt, wie dies beispielsweise in Fig. 3 durch die gestrichelte Linie angedeutet ist, so bedeutet dies in der Konsequenz, dass das Meniskusteil dann zwar entlang des Lenkers translatorisch verschiebbar ist (in anteriorer/posteriorer Richtung in Fig. 2), jedoch nach wie vor nicht drehbar ist.

Will man lediglich eine Drehbarkeit des Meniskusteils erreichen ohne eine translatorische Verschiebbarkeit des Meniskusteils auf dem Tibiateil zu ermöglichen, so wähle man das Führungselement 6b gemäss Fig. 4. Dieses Führungselement 4b ist von der Länge L und der Breite B her wieder auf die Länge und Breite des Langlochs abgestimmt, sodass keine translatorische Verschiebung des Meniskusteils relativ zum Tibiateil möglich ist, sehr wohl jedoch eine Drehung. Der Zapfen 63b weist nämlich keinen Vorsprung auf, sodass der Zapfen 63b in der Bohrung des Tibiateils drehbar gelagert ist. Die Drehbarkeit des Zapfens 63b in der Bohrung des Tibiateils hat aber zur Folge, dass das Führungselement 6b und dadurch letzlich auch das Meniskusteil gegenüber dem Tibiateil drehbar ist.

Schliesslich ist noch der Fall zu betrachten, bei dem sowohl eine Drehbarkeit als auch eine translatorische Verschiebbarkeit des Meniskusteils relativ zum Tibiateil erwünscht ist, was insbesondere bei intaktem Bandapparat in Betracht kommt. Hierzu wähle man erneut das Führungselement 6b aus Fig.4, jedoch mit einer geringeren Länge, wie dies durch die gestrichelte Linie in Fig. 4 angedeutet ist. Das Führungselement 6b ist dann gegenüber dem Tibiateil drehbar, wodurch auch das Meniskusteil gegenüber dem Tibiateil drehbar ist. Durch die geringere Länge des Lenkers 60b gegenüber dem Langloch des Meniskusteils ist das Meniskusteil auch entlang dem Lenker 60b translatorisch verschiebbar.

Was die translatorische Verschiebbarkeit betrifft, so ist es klar, dass man grundsätzlich auch das Meniskusteil auswechseln kann anstatt das Führungselement auszuwechseln. Das Langloch des Meniskusteils muss dann in seinen Abmessungen so gewählt werden, dass es entweder auf die Länge des Lenkers abgestimmt ist (keine translatorische Verschiebung möglich) oder dass es länger ist als der Lenker (translatorische Verschiebung möglich).

Was die Drehung betrifft, so ist es auch möglich, durch eine Variation der Breite des Vorsprungs 630a (Fig. 3) des Zapfens 63a relativ zur Breite der Aussparung 130a der Bohrung 13a eine begrenzte Drehbarkeit zuzulassen, wenn dies erwünscht ist.

Insgesamt lässt sich also feststellen, dass mit ein- und demselben Tibiateil praktisch sämtliche Arten des Spektrums der Bewegbarkeit des Meniskusteils relativ zum Tibiateil abgedeckt werden kann. Dieses Spektrum der Bewegbarkeit reicht von einem relativ zum Tibiateil unbeweglichen Meniskusteil bis hin zu einem relativ zum Tibiateil sowohl drehbaren als auch translatorisch verschiebbaren Meniskusteil. Erreicht werden kann dies auf einfache Weise durch die Bereitstellung mehrerer verschiedener Führungselemente, die bei zusammengesetzter Prothese sowohl mit dem Tibiateil als auch mit dem Meniskusteil in Eingriff sind. Alternativ können - was die translatorische Verschiebbarkeit betrifft - verschiedene Meniskusteile bereitgestellt werden, die ein unterschiedlich langes Langloch aufweisen.

Für die Materialien der einzelnen Teile kommen an sich bekannte Materialien in Frage. Was das Tibiateil betrifft, so kann dieses grundsätzlich aus einer Kobalt-Chrom-Legierung hergestellt sein. Sofern eine zementfreie Implantation erfolgen soll, können die mit dem Knochen in Kontakt kommenden Teile, insbesondere die Unterseite des Tibiateils, das Ansatzstück, die Verankerungsstifte etc., mit einer porösen Titanschicht überzogen sein, welche für das Einwachsen besonders geeignet ist. Das Meniskusteil ist beispielsweise aus Polyethylen hergestellt, insbesondere aus ultrahochmolekularem Polyethylen, welches besonders gute (nämlich praktisch keine) Verschleisseigenschaften aufweist. Das Führungselement kann wiederum aus einer Kobalt-Chrom-Legierung hergestellt sein, was ebenso für das Stabilisierungselement gilt und auch für das Femurteil. Bei zementfreien Implantationen können diejenigen Teile des Femurteils, die mit dem Knochen in Kontakt kommen, ebenfalls mit einer porösen Titanschicht überzogen sein.

## Patentansprüche

1. Bausatz für eine Kniegelenkprothese, mit einem Tibiateil (1,1a), einem Femurteil (4) sowie mit einem zwischen dem Femurteil (4) und dem Tibiateil (1,1a) anzuordnenden Meniskusteil (3,3a), welcher Bausatz die Zusammenstellung von verschiedenen Prothesentypen erlaubt, nämlich von solchen Prothesen, bei denen das Meniskusteil (3a) relativ zum Tibiateil (1a) unbeweglich angeordnet ist, sowie von solchen Prothesen, bei denen das Meniskusteil (3) relativ zum Tibiateil (1) bewegbar angeordnet ist, wobei der Bausatz mehrere Führungselemente (6,6a,6b) umfasst, die so ausgebildet sind, dass das jeweilige Führungselement (6,6a,6b) bei zusammengesetzter Prothese mit dem Tibiateil (1,1a) und mit dem Meniskusteil (3,3a) in Eingriff ist und die Bewegbarkeit des Meniskusteils (3,3a) relativ zum Tibiateil (1,1a) festlegt, wobei das Tibiateil (1,1a) in seiner dem Femurteil (4) zugewandten Fläche (12,12a) eine Bohrung (13,13a) aufweist, die mit einer Aussparung (130a) versehen ist, wobei die einzelnen Führungselemente (6,6a,6b) jeweils einen Zapfen (63,63a,63b) aufweisen, der bei zusammengesetzter Prothese in diese Bohrung (13,13a) des Tibiateils (1,1a) hineinragt, sowie einen Lenker (60,60a,60b), der bei zusammengesetzter Prothese in Eingriff mit einer entsprechenden Führungsfläche am Meniskusteil ist, und wobei manche Zapfen (63a) einen Vorsprung (630a) aufweisen, der in der Aussparung (130a) der Bohrung (13a) zu liegen kommt, während andere Zapfen (6,6b) keinen solchen Vorsprung aufweisen.

2. Bausatz nach Anspruch 1, bei welchem das Meniskusteil (3,3a) ein Langloch (30,30a) aufweist, in welchem der Lenker (60,60a,60b) bei zusammengesetzter Prothese aufgenommen wird.

3. Bausatz nach einem der Ansprüche 1 oder 2, bei welchem Führungselemente (6,6a,6b) mit unterschiedlicher Länge (L) des Lenkers (60,60a,60b) vorgesehen sind, sodass bei ein und demselben Meniskusteil (3,3a) je nach Länge des Lenkers (60,60a,60b) bei zusammengesetzter Prothese eine translatorische Bewegung des Meniskusteils (3,3a) möglich ist oder nicht.

4. Bausatz nach einem der Ansprüche 1 bis 3, bei welchem der Zapfen (63,63a,63b) des Führungselements (6,6a,6b) als Hohlzapfen ausgebildet ist zur Aufnahme eines Zapfens (71) eines Stabilisierungselementes (7), welches bei zusammengesetzter Prothese zwischen die Kondylen (41) des Femurteils (4) hineinragt und Mittel (70) zur Varus-Valgus-Stabilisierung aufweist.

## Claims

1. A kit for a knee joint prosthesis, comprising a tibia part (1, 1a), a femur part (4) and a meniscus part (3, 3a) to be arranged between the femur part (4) and the tibia part (1, 1a), said kit enabling the assembly of different prosthesis types, namely of such prostheses in which the meniscus part (3a) is arranged immobile relative to the tibia part (1a) as well as of such prostheses in which the meniscus part (3) is arranged so as to be movable relative to the tibia part (1), wherein the kit includes a plurality of guiding elements (6, 6a, 6b) which are formed such that the respective guiding element (6, 6a, 6b) is in engagement with the tibia part (1, 1a) and with the meniscus part (3, 3a) when the prosthesis is assembled and determines the movability of the meniscus part (3, 3a) relative to the tibia part (1, 1a); wherein the tibia part (1, 1a) has a bore (13, 13a) in its surface (12, 12a) facing the femur part (4) which is provided with a cut-out (130a); wherein the individual guiding elements (6, 6a, 6b) each have a pin (63, 63a, 63b) which protrudes into this bore (13, 13a) of the tibia part (1, 1a) when the prosthesis is assembled and a guiding part (60, 60a, 60b) which is in engagement with a corresponding guiding surface at the meniscus part when the prosthesis is assembled; and wherein some of the pins (63a) have a projection (630a) which comes to lie in the cut-out (130a) of the bore (13a), whereas other pins (6, 6b) have no projections of this kind.

2. A kit in accordance with claim 1, wherein the meniscus part (3, 3a) has an elongate hole (30, 30a) in which the guiding part (60, 60a, 60b) is received when the prosthesis is assembled.

3. A kit in accordance with one of the claims 1 or 2, wherein guiding elements (6, 6a, 6b) with different lengths (L) of the guiding part (60, 60a, 60b) are provided so that for one and the same meniscus part (3, 3a), depending on the length of the guiding part (60, 60a, 60b), a translational movement of the meniscus part (3, 3a) is possible or not when the prosthesis is assembled.

4. A kit in accordance with any one of the claims 1 to 3, wherein the pin (63, 63a, 63b) of the guiding element (6, 6a, 6b) is formed as a hollow pin for receiving a pin (71) of a stabilizing element (7) which protrudes in between the condyles (41) of the femur part (4) when the prosthesis is assembled and has means (70) for the varus-valgus stabilizing.

## Revendications

1. Jeu de pièces pour une prothèse d'articulation du genou, comprenant une pièce formant tibia (1, 1a), une pièce formant fémur (4) ainsi qu'une pièce formant ménisque (3, 3a) destinée à être agencée entre la pièce formant fémur (4) et la pièce formant tibia (1, 1a), ledit jeu de pièces permettant l'assemblage de différents types de prothèses, à savoir des prothèses dans lesquelles la pièce formant ménisque (3a) est agencée de façon immobile par rapport à la pièce formant tibia (1a) ainsi que des prothèses dans lesquelles la pièce formant ménisque (3) est agencée de façon mobile par rapport à la pièce formant tibia (1),
dans lequel le jeu de pièces comprend plusieurs éléments de guidage (6, 6a, 6b) qui sont réalisés de telle manière que l'élément de guidage respectif (6, 6a, 6b) est en engagement, lorsque la prothèse est assemblée, avec la pièce formant tibia (1, 1a) et avec la pièce formant ménisque (3, 3a) et détermine la mobilité de la pièce formant ménisque (3, 3 a) par rapport à la pièce formant tibia (1, 1a),
dans lequel la pièce formant tibia (1, 1a) comporte, dans sa surface (12, 12a) tournée vers la pièce formant fémur (4), un perçage (13, 13a) qui est pourvu d'une échancrure (130a), dans lequel les éléments de guidage individuels (6, 6a, 6b) comportent chacun un tenon (63, 63a, 63b) qui, lorsque la prothèse est assemblée, pénètre dans ce perçage (13, 13a) de la pièce formant tibia (1, 1a), ainsi qu'un organe directeur (60, 60a, 60b) qui, lorsque 1a prothèse est assemblée, est en engagement avec une surface de guidage correspondante sur la pièce formant ménisque, et dans lequel certains tenons (63a) présentent une saillie (630a) qui vient se placer dans l'échancrure (130a) du perçage (13a), alors que d'autres tenons (6, 6b) ne présentent pas de telles saillies.

2. Jeu de pièces selon la revendication 1, dans lequel la pièce formant ménisque (3, 3a) comporte un trou oblong (30, 30a) dans lequel est reçu l'organe directeur (60, 60a, 60b) lorsque la prothèse est assemblée.

3. Jeu de pièces selon l'une des revendications 1 ou 2, dans lequel des éléments de guidage (6, 6a, 6b) avec des organes directeurs (60, 60a, 60b) de longueurs différentes (L) sont prévus, de sorte qu'avec une seule et même pièce formant ménisque (3, 3a), selon la longueur de l'organe directeur (60, 60a, 60b) lorsque la prothèse est assemblée, un mouvement de translation de la pièce formant ménisque (3, 3a) est possible ou non.

4. Jeu de pièces selon l'une des revendications 1 à 3, dans lequel le tenon (63, 63a, 63b) de l'élément de guidage (6, 6a, 6b) est réalisé sous forme de tenon creux pour recevoir un tenon (71) d'un élément de stabilisation (7) qui, lorsque la prothèse est assemblée, pénètre entre les condyles (41) de la pièce formant fémur (4) et qui présente des moyens (70) pour la stabilisation "varus-valgus".
